# EUROPEAN PATENT APPLICATION

(11) **EP 2 578 188 A1**
(43) Date of publication of application: **10.04.2013**
(21) Application number: 11184407.2
(22) Date of filing: 07.10.2011
(51) Int. Cl.: A61F 9/00, A61M 5/32

(54) **Apparatus for intraocular injection**

(71) Applicant: Sanofi-Aventis Deutschland GmbH, 65929 Frankfurt (DE)
(72) Inventor: The designation of the inventor has not yet been filed
(74) Representative: KEIL & SCHAAFHAUSEN Patentanwälte

(57) **Abstract**

Described is an apparatus for intraocular injection comprising a body (10) adapted to accommodate a syringe (12) having a needle (14), a needle cover (16) adapted to cover the needle (14), and two or more resilient arms (20, 21) extending distally from the body (10). The body (10) has a longitudinal axis. The arms (20, 21) are adapted to selectively engage the needle cover (16).

## Description

The present invention relates to an apparatus for intraocular injection.

### Background

An intraocular injection device may be used to administer therapeutic substances to eyes, such as eyes of mammals having eye disorders or diseases.

A number of vision-threatening disorders or diseases of the eye need to deliver a medicament (pharmaceutical, biological, etc.) and/or implantable device to a posterior segment of the eye by intraocular delivery (more specifically intravitreal delivery). One such technique for intraocular delivery is accomplished by intraocular injection into the vitreous body.

A conventional apparatus for intraocular injection may include a pre-filled syringe of a medicament. A conventional pre-filled syringe is supplied with a needle cover in order to maintain sterility of a needle. However, the needle cover is typically frictionally held on the needle which can result in dislodging of the needle cover. If any portion of the needle becomes unsterile prior to use, the syringe must be discarded.

Therefore, there is a need for an apparatus for intraocular injection which ensures that a needle remains covered until use and facilitates removal of a needle cover.

### Summary

The exemplary embodiments of the present invention describe an apparatus for intraocular injection.

In an exemplary embodiment, an apparatus for intraocular injection according to the present invention comprises a body adapted to accommodate a syringe having a needle, a needle cover adapted to cover the needle, and two or more resilient arms extending distally from the body. The body has a longitudinal axis. The arms are adapted to selectively engage the needle cover.

In an exemplary embodiment, in a first configuration, the arms engage the needle cover. The arms are deflected away from the longitudinal axis into a second configuration when the needle cover as the needle cover is removed from the needle. When the needle cover has been removed from the needle, the arms move radially toward the longitudinal axis to a third configuration.

In an exemplary embodiment, the arms are coupled to the body via respective hinges.

In an exemplary embodiment, the arms are formed as a section, for example a longitudinal section, of a cylinder or a hollow cone or hollow double cone.

In an exemplary embodiment, inner rims of the arms are adapted to engage grooves on the needle cover.

In an exemplary embodiment, the arms include a placement foot.

The person skilled in the art understands that the present invention is not restricted to the explained possibilities.

The above mentioned advantages as well as other advantages of various aspects of the present invention will become apparent to those of ordinary skill in the art by reading the following detailed description, with appropriate reference to the accompanying drawings.

### Brief Description of the Drawings

Exemplary embodiments of the present invention are described herein with reference to the schematic drawings in which:
Fig. 1 and 2 illustrate a side view and a perspective view of a cross section of an exemplary embodiment of an apparatus for intraocular injection in a first configuration;
Fig. 3 and 4 show the exemplary embodiment of an apparatus for intraocular during removal of a needle cover in a second configuration; and
Fig. 5 and 6 depict the exemplary embodiment of an apparatus for intraocular in a third configuration after removal of the needle cover.

### Detailed Description

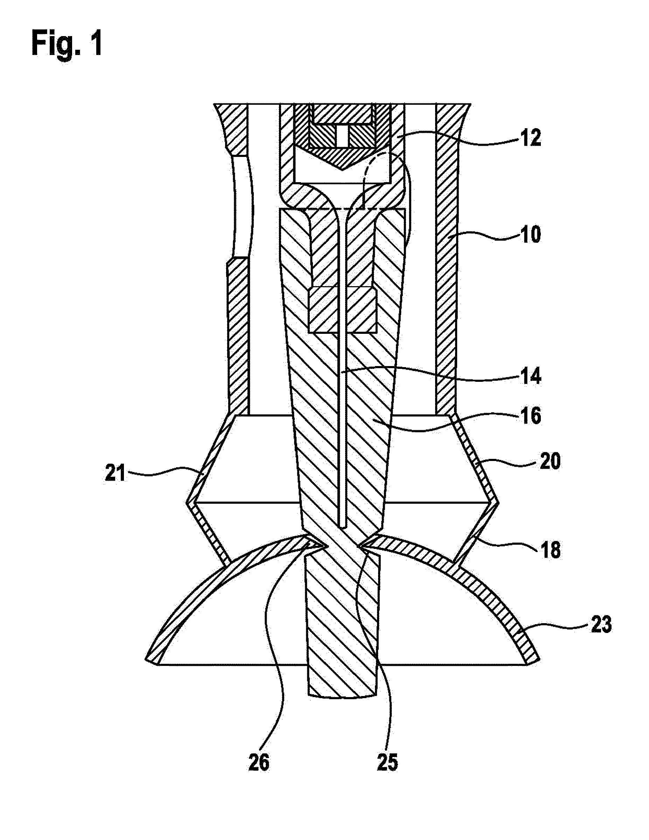

Figures 1 to 6 illustrate an exemplary embodiment of an apparatus for intraocular injection according to the present invention. As shown in the exemplary embodiment of Figure 1, the apparatus comprises a hollow body 10 which is adapted to accommodate a pre-filled syringe 12 having a needle 14 at its distal end. In the exemplary embodiment, the apparatus may be an auto-injector, delivering the entire contents of the syringe 12 when the apparatus is activated. In another exemplary embodiment, the apparatus may be a reusable, fixed dose delivery device for administering only a portion of the contents of syringe 12 per injection. Those of skill in the art will understand that in another exemplary embodiment the pre-filled syringe 12 may be replaced by a medicament cartridge having a needle or having an interface for engaging a removable needle assembly.

In an exemplary embodiment, a housing sleeve 18 may be disposed at a distal end of the body 10. The housing sleeve 18 may be composed of two arms 20 and 21. The person skilled in the art understands that the housing sleeve 18 may comprise more than two parts or may be made in one piece as well.

In an exemplary embodiment, each of the arms 20, 21 of the housing sleeve 18 is connected to the distal end of the body 10 by a hinge, allowing for motion of the arms 20, 21 in at least one plane relative to the body 10. The hinge may be a joint which couples the arms 20, 21 to the body 10, or the hinge may be a living hinge formed at a junction of the integrally formed arms 20, 21 and body 10.

In an exemplary embodiment, distal ends of the arms 20, 21 forms a placement foot 23 adapted for placement on a target anatomical structure, e.g., the eye. For example, as shown in the exemplary embodiments in Figures 1 to 6, the placement foot 23 takes the form of a semi-circle for placement on the eye, and the distal ends of each of the arms 20, 21 forms one half of the semi-circle. Those of skill in the art will understand that the placement foot 23 may take any other suitable form.

In an exemplary embodiment, a needle cover 16 is disposed on the needle 14. The needle cover 16 may cover an entire length of needle 14 to maintain sterility. In an exemplary embodiment, the needle cover 16 includes one or more grooves 25 which are adapted to receive a rim 26 formed on a medial edge of each of the distal ends of the arms 20, 21. In another exemplary embodiment, the rim 26 may include a barb for engaging the needle cover 16, and the needle cover 16 may not include the grooves 25.

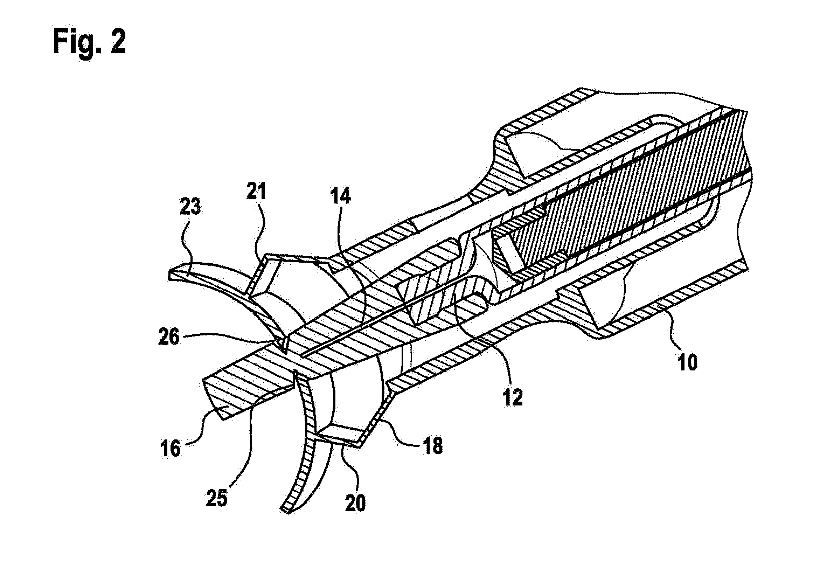

Figures 1 and 2 show the apparatus in a first configuration in which the rims 26 on the arms 20, 21 engage the grooves 25 on the needle cover 16. In an exemplary embodiment, the arms 20, 21 are radially biased toward a longitudinal axis of the body 10 in the first configuration. For example, the hinge formed between the arms 20, 21 and the body 10 may be spring-loaded (or utilize a resilient effect) to bias the arms 20, 21 in the first configuration so that the rims 26 engage the needle cover 16, if present.

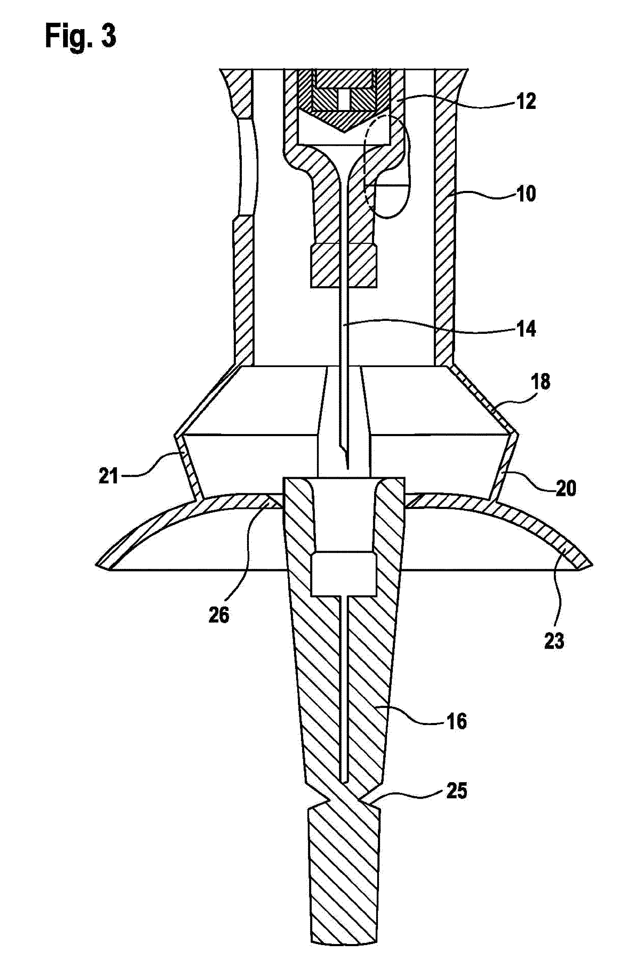

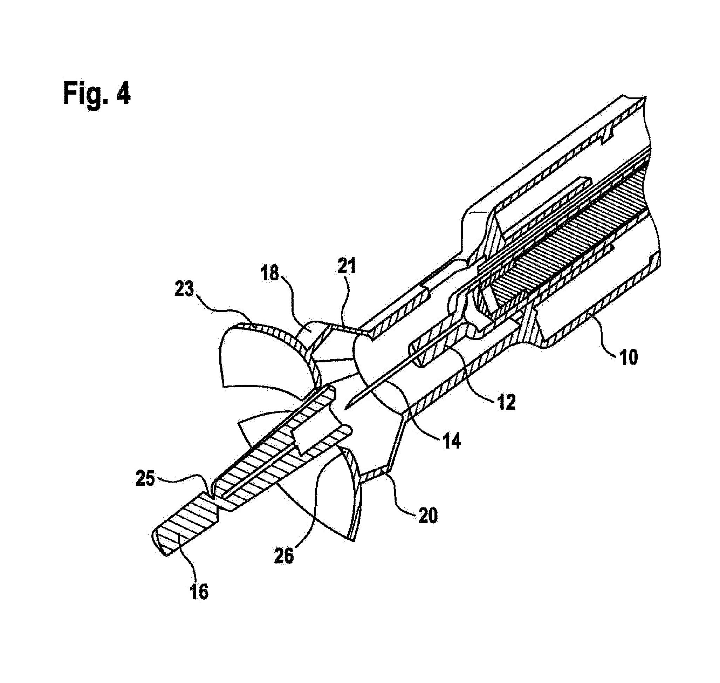

Figures 3 and 4 show the apparatus in a second configuration in which the arms 20, 21 are deflected radially away from the longitudinal axis of the body 10 and the rims 26 disengage the needle cover 16. In the second configuration, the needle cover 16 can be removed from the needle 14. In an exemplary embodiment, the arms 20, 21 may be oriented in the second configuration by pulling the needle cover 16 distally through the arms 20, 21, which are deflected radially away from the longitudinal axis of the body 10 against the biasing force. In this exemplary embodiment, the grooves 25 may have ramped portions adapted to engage corresponding ramped portions on the rims to facilitate movement of the arms 20, 21 from the first configuration to the second configuration. In another exemplary embodiment, either or both of the arms 20, 21, the housing sleeve 18, or the body 10 may include a handle, lever, gear, etc. which, when actuated, positions the arms 20, 21 in the second configuration.

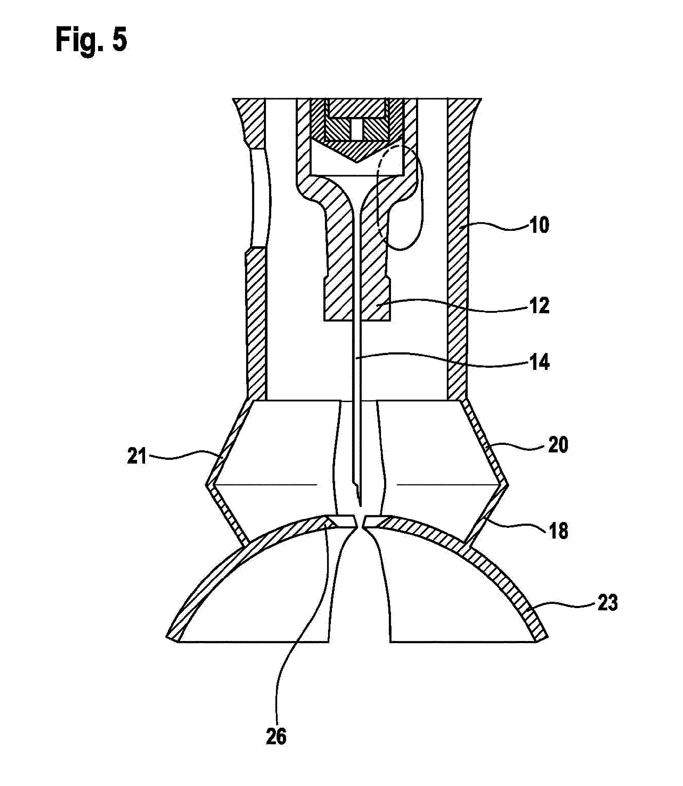

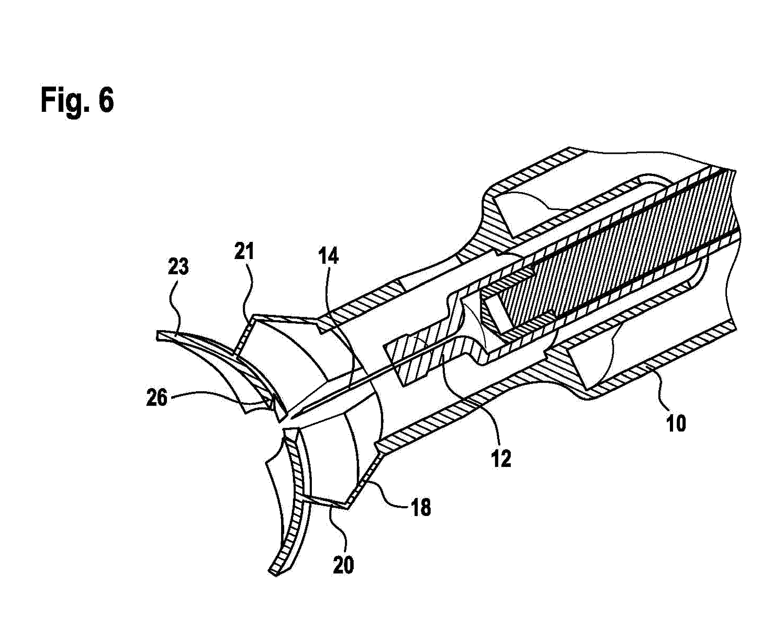

Figures 5 and 6 show the apparatus in a third configuration, after the needle cover 16 has been removed from the needle 14. Due to the biasing force, the arms 20, 21 have returned to a non-deflected configuration. Though the needle cover 16 has been removed, the needle 14 remains shielded by the housing sleeve 18 and the arms 20, 21, which may prevent needle stick injuries prior to and after injection.

After the needle cover 16 has been removed, the apparatus can be placed on the eye and positioned properly using the placement foot 23 to administer a medicament contained within the syringe 12. In an exemplary embodiment, when the apparatus is activated, the syringe 12 moves distally within the body 10 so the needle 14 projects distally from the placement foot 23 and into the eye. After the injection is administered, a spring or other mechanism may be used to retract the needle 14 and/or the syringe 12 into the body 10 such that the needle 14 is again shielded by the housing sleeve 18 and the arms 20, 21.

Those of skill in the art will understand that the placement foot 23 of the exemplary embodiments may be made from an at least partially transparent material such that alignment with the eye, e.g. a periphery of the cornea, may be facilitated. Further, those of skill in the art will understand that an underside of the placement foot 23, for example a surface of the foot 23 which contacts the eye may include a frictional layer or other means for gripping, without injury, the eye.

When the apparatus has been properly placed on the eye, the physician may depress a plunger or similar depressable element coupled to the body 10 and/or the syringe 12 which advances the syringe 12 distally within the body 10 towards the injection site. Then a medicament may be delivered to the predetermined region of the eye.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. An apparatus for intraocular injection comprising:
a body (10) adapted to accommodate a syringe (12) having a needle (14),
wherein the body (10) has a longitudinal axis;
a needle cover (16) adapted to cover the needle (14); and
two or more resilient arms (20, 21) extending distally from the body (10), the arms (20, 21) adapted to selectively engage the needle cover (16).

2. The apparatus according to claim 1 wherein, in a first configuration, the arms (20, 21) engage the needle cover (16).

3. The apparatus according to claim 2 wherein the arms (20, 21) are deflected away from the longitudinal axis into a second configuration when the needle cover (16) as the needle cover (16) is removed from the needle (14).

4. The apparatus according to claim 3 wherein, when the needle cover (16) has been removed from the needle (14), the arms (20, 21) move radially toward the longitudinal axis to a third configuration.

5. The apparatus according to any of the preceding claims, wherein the arms (20, 21) are coupled to the body (10) via respective hinges.

6. The apparatus according to any of the preceding claims wherein the arms (20, 21) are formed as a section, for example a longitudinal section, of a cylinder or a hollow cone or hollow double cone.

7. The apparatus according to any of the preceding claims wherein inner rims (26) of the arms (20, 21) are adapted to engage grooves (25) on the needle cover (16).

8. The apparatus according to any of the preceding claims wherein the arms (20, 21) include a placement foot (23).
